# EUROPÄISCHE PATENTSCHRIFT

(11) **EP 2 559 410 B1**
(45) Veröffentlichungstag und Bekanntmachung des Hinweises auf die Patenterteilung: **25.12.2013**
(21) Anmeldenummer: 11006745.1
(22) Anmeldetag: 18.08.2011
(51) Int. Cl.: A61F 5/00

(54) **Einrichtung zur Behandlung von Fettleibigkeit**
Device for treating obesity
Dispositif de traitement de l'obésité

(43) Veröffentlichungstag der Anmeldung: 20.02.2013
(73) Patentinhaber: A.M.I. Agency for Medical Innovations GmbH, 6800 Feldkirch (AT)
(72) Erfinder: Super, Paul, Hampton in Arden Silihull West Midlands B92 0HE (GB)
(74) Vertreter: Hofmann, Ralf U.

(56) Entgegenhaltungen:
- EP-A1- 1 669 045
- WO-A1-2011/071834
- AT-A1- 504 158
- US-A1- 2005 119 674
- US-A1- 2006 015 151

## Beschreibung

Die Erfindung bezieht sich auf eine Einrichtung zur Behandlung von Fettleibigkeit, umfassend ein ringförmig um den Magen legbares Bandteil mit einer Verschlusseinrichtung, mit der das Bandteil zu einem umfangsgeschlossenen Ring verschließbar ist, der eine Öffnung mit einer zentralen Achse aufweist, und ein mit dem Bandteil verbundenes und von diesem auf einer Seite abstehendes Stützteil zum Abstützen eines gegenüber dem angelegten Bandteil proximal liegenden Magenbereichs.

Bekannt sind Magenbänder für die Behandlung der Fettleibigkeit, bei denen ein Bandteil ringförmig um den Magen gelegt wird, um durch das Einschnüren des Magens eine Engstelle für die Nahrungspassage durch den Magen zu schaffen. Meist weisen diese Magenbänder einen umlaufenden, mit einem Füllmedium befüllbaren Hohlraum auf, sodass durch Einfüllen des Füllmediums die Größe der Öffnung des Magenbandes variiert werden kann, um die Einschnürung des Magens einzustellen.

Die aufgenommene Nahrung staut sich vor dieser chirurgisch geschaffenen Engstelle und der Druck im gestauten Magenanteil steigt, wodurch es zu einer Vorwölbung der Magenvorderwand in die Bauchhöhle kommt. Diese übermäßige Vorwölbung der Magenvorderwand führt nach längerer Zeit zu einer unerwünschten Erweiterung des abgeschnürten Magenteils, wodurch der Erfolg der operativen Behandlung der Fettleibigkeit beeinträchtigt wird.

Um diesen Nachteil zu überwinden ist aus der EP 1669045 A1 eine Einrichtung der eingangs genannten Art bekannt. Das am Bandteil angebrachte, von diesem auf einer Seite abstehende Stützteil wird zusammen mit dem Bandteil um den Magen gelegt und nach dem Schließen des Bandteils zu einem Ring werden die einander nunmehr benachbart liegenden umfänglichen Enden des Stützteils miteinander verbunden, wodurch eine geschlossene Hülle ausgebildet wird, die einen proximal des Bandteils liegenden Magenteil umfänglich umgibt.

Weiters zeigt die AT 504158 B1 eine Einrichtung mit zwei oder mehr Bandteilen, um den Magen mehrstufig einzuschnüren. Zwischen den Bandteilen erstrecken sich an den einander zugewandten Seitenrändern der Bandteile angebrachte Hüllteile, die mit den Bandteilen um den Magen gelegt werden und im angelegten Zustand der Einrichtung den Magen umhüllen.

Aus der US 2005/0119674 A1 und WO 02/053040 A1 gehen weitere Hüllvorrichtungen in Form von Bändern, Netzen und dergleichen hervor, welche umfänglich um den Magen angelegt werden.

Aus der US 6,165,122 A ist weiters eine gelochte elastische Hülle bekannt, die umfänglich um den Herzmuskel gelegt wird, um dessen unerwünschte Erweiterung zu verhindern.

Um die den Magen umhüllenden vorbekannten Einrichtungen anzulegen, ist eine weite ausführliche chirurgische Trennung der Magenhinterwand von der Hinterwand der Bauchhöhle erforderlich. Dies führt zu einem stark erhöhten Operationsrisiko für den Patienten. Es kann beim Durchtrennen der Gewebeschichten zu unerwünschten starken Blutungen kommen, eine längere Operationszeit wird benötigt und das A-nästhesierisiko bei dieser besonders kritischen Patientengruppe von fettleibigen Personen wird erhöht. Weiters bleibt eine wesentlich größere Wundfläche in der Bauchhöhle zurück, verbunden mit erhöhter Absonderung von Wundsekret, was wiederum den Einsatz von Ablaufkanülen erforderlich macht. Diese medizinischen Gründe sind Ursache dafür, dass sich die bekannten technischen Lösungen von umfänglichen elastischen Schutzvorrichtungen gegen eine unerwünschte Vergrößerung der chirurgisch eingeschnürten Magenabschnitte in der chirurgischen Praxis nicht durchgesetzt haben.

Aufgabe der Erfindung ist es eine Einrichtung der eingangs genannten Art bereitzustellen, durch die einer Ausdehnung des Magens im gestauten Bereich entgegengewirkt wird, wobei das Operationsrisiko beim Anlegen verringert wird. Erfindungsgemäß gelingt dies durch eine Einrichtung mit den Merkmalen des Anspruchs 1.

Bei der Einrichtung gemäß der Erfindung erstreckt sich das Stützteil in der Schnittebene eines jeden durch das Stützteil verlaufenden Querschnitts, der parallel zur Mittelebene liegt, in welcher das zu einem Ring verschlossene Bandteil liegt und welche mittig durch das Bandteil verläuft, über weniger als 270° um die zentrale Achse der Öffnung des Bandteils. Es liegen also, bezogen auf ein Polarkoordinatensystem, das in der Ebene des Querschnitts durch das Stützteil liegt und die Mittelachse als Ursprung aufweist, alle Punkte des Stützteils in einem Winkelbereich von weniger als 270°. Über den restlichen Winkelbereich von mehr als 90° erstreckt sich das Stützteil somit nicht, d.h. in diesem Winkelbereich liegen keine Punkte des Stützteils. Das Stützteil ist also umfangsoffen ausgebildet mit einer Umfangserstreckung von weniger als 270° über seine gesamte Ausdehnung in die Richtung der zentralen Achse.

Durch diese Ausbildung ist eine chirurgische Trennung der Magenhinterwand von der Hinterwand der Bauchhöhle nur soweit erforderlich, als ein Durchgang (Kanal) für die Durchführung des Magenbandes selbst benötigt wird. Das Stützteil, das im angelegten Zustand den proximal des Bandteils liegenden Teil des Magens im Bereich der Magenvorderwand überdeckt, muss nicht um die Magenhinterwand herumgeführt werden. Um dennoch eine stabile Abstützung des gestauten Magenteils zu erreichen, kann das Stützteil vorteilhafterweise an vom Bandteil beabstandeten Stellen beidseitig (=lateral) des abgestützten Magenbereichs mit der Hinterwand der Bauchhöhle verbunden werden, insbesondere durch Vernähen. Solche Verbindungspunkte mit der Hinterwand der Bauchhöhle liegen vorzugsweise in Bereichen, an denen ein Seitenrand des Stützteils, der am vom Bandteil entfernten Ende des Stützteils die Erstreckung des Stützteils in Richtung der zentralen Achse begrenzt, mit den Längsrändern des Stützteils zusammentrifft, die die Erstreckung des Stützteils um die zentrale Achse begrenzen. An den Verbindungspunkten ist das Stützteil vorteilhafterweise verstärkt ausgebildet, z.B. durch Gewebeeinlagen. Vorzugsweise sind an diesen Verbindungs- bzw. Übergangsbereichen zwischen dem Längsrand und dem jeweiligen Seitenrand am Stützteil Nähösen ausgebildet. Zusätzliche, vorzugsweise verstärkte, Verbindungspunkte, insbesondere Nähösen, mit der Hinterwand der Bauchhöhle können vorgesehen sein, insbesondere im Bereich der Längsränder des Stützteils. Zusätzlich können auch Verbindungspunkte zur Verbindung des Stützteils mit der Magenwand vorgesehen sein, wobei die Verbindung vorzugsweise wiederum durch Vernähen erfolgt, beispielsweise im Bereich des Seitenrandes des Stützteils.

Durch die erfindungsgemäße Ausbildung gelingt es, die Vorteile von herkömmlichen Magenbändern ohne Stützteile, welche mit einer relativ gering invasiven Operation angelegt werden können, und von Magenbändern mit Stützteilen, die einer Aufweitung des gestauten Magenteils entgegenwirken, zu verbinden.

In einer vorteilhaften Ausführungsform der Erfindung ist im Bereich des Stützteils, der in einem Abstand von der Mittelebene liegt, welcher mehr als zwei Drittel des maximalen Abstands des Seitenrands des Stützteils von der Mittelebene beträgt, die größte Erstreckung des Stützteils um die zentrale Achse kleiner als die größte Erstreckung des Stützteils um die zentrale Achse im Bereich des Stützteils, der in einem Abstand von der Mittelebene liegt, welcher weniger als ein Drittel des maximalen Abstands des Seitenrands des Stützteils von der Mittelebene beträgt. Die Erstreckung des Stützteils um die zentrale Achse in einem bestimmten Abstand von der Mittelebene gibt hierbei den Winkelbereich an, über den das Stützteil in einem in diesem Abstand von der Mittelebene liegenden Querschnitt verläuft.

Durch diese Ausbildung wird eine gute Abstützung des gestauten Magenteils erreicht, wobei das Stützteil so an der Hinterwand der Bauchhöhle befestigbar ist, dass das Stützteil seine Abstützfunktion zuverlässig erfüllt.

Günstigerweise ist das Stützteil mit einer Vielzahl von über seine Ausdehnung verteilten Durchtrittsöffnungen ausgebildet, wobei die Summe der Flächen der Durchtrittsöffnungen vorzugsweise einen Großteil der Gesamtfläche des Stützteils ausmacht. Besonders bevorzugt ist das Stützteil netzartig ausgebildet.

Die Verschlusseinrichtung zum Verschließen des Bandteils zu einem umfangsgeschlossenen Ring kann in herkömmlicher Weise ausgebildet sein. Insbesondere können hierzu im Bereich der beiden Bandenden angeordnete, miteinander verbindbare Verschlussteile vorhanden sein. In einer vorteilhaften Ausführungsform weist ein erstes der beiden Verschlussteile in bekannter Weise einen Rastvorsprung auf und das zweite der beiden Verschlussteile weist eine Durchtrittsöffnung auf, durch die das erste Verschlussteil durchziehbar ist, wobei der Rastvorsprung des ersten Verschlussteils hinter der Durchtrittsöffnung mit dem zweiten Verschlussteil verrastet.

Zur Verbindung des Stützteil mit dem Bandteil ist das Stützteil günstigerweise am Bandteil angeklebt, vorzugsweise im Bereich eines der Längsränder des Bandteils.

Zur umfangsoffenen Ausbildung des Stützteils erstreckt sich das Stützteil im Verbindungsbereich mit dem Bandteil nicht über die gesamte Länge des Bandteils (d.h. nicht über den gesamten Umfang des zu einem Ring geschlossenen Bandteils). Vorzugsweise ist ein erster Abschnitt der Längserstreckung des Bandteils vorgesehen, der an ein erstes Ende des Bandteils anschließt und dessen Länge zumindest ein Drittel der gesamten Länge des Bandteils beträgt, vorzugsweise zumindest zwei Fünftel der gesamten Länge des Bandteils, wobei über diesem ersten Abschnitt der Längserstreckung des Bandteils kein von einer Seite des Bandteils abstehendes Stützteil vorgesehen ist. Das Stützteil steht nur über einen zweiten Abschnitt der Längserstreckung des Bandteils vom Bandteil seitlich ab, wobei der zweite Abschnitt der Längserstreckung des Bandteils an das zweite Ende des Bandteils anschließt und sich über die Längserstreckung des Bandteils erstreckt, über welche sich der erste Abschnitt des Bandteils nicht erstreckt.

Dadurch, dass der erste Abschnitt der Längserstreckung des Bandteils frei von seitlich abstehenden Stützteilen ausgebildet ist, kann der erste Abschnitt des Bandteils durch einen relativ kleinen Kanal durchgezogen werden, der zwischen der Hinterwand des Magens und der Hinterwand der Bauchhöhle freipräpariert worden ist, durchgezogen werden, sodass dieser erste Abschnitt sich um die Hinterwand des Magens erstreckt. In der Folge kann die Verschlusseinrichtung, die vorteilhafterweise Verschlussteile an beiden Enden des Bandteils aufweist, geschlossen werden. Das Stützteil liegt somit im Bereich der Vorderwand des Magens und kann, wie bereits erwähnt, in der Folge in vom Bandteil entfernt gelegenen Bereichen beidseitig lateral des Magens an der Hinterwand der Bauchhöhle befestigt werden.

Weitere Vorteile und Einzelheiten der Erfindung werden im Folgenden anhand der beiliegenden Zeichnung erläutert. In dieser zeigen:
Fig. 1 ein Ausführungsbeispiel einer erfindungsgemäßen Einrichtung im geschlossenen Zustand des Bandteils, in Schrägsicht;
Fig. 2 und Fig. 3 die Einrichtung von Fig. 1 in Schrägsichten aus sich unterscheidenden Blickrichtungen;
Fig. 4 eine Seitenansicht der Einrichtung von Fig. 1;
Fig. 5 eine Seitenansicht von Fig. 1 aus einer anderen Blickrichtung;
Fig. 6 einen Schnitt entlang der Linie AA von Fig. 5;
Fig. 7 einen Schnitt entlang der Linie BB von Fig. 5;
Fig. 8 einen Schnitt entlang der Linie CC von Fig. 5;
Fig. 9 einen Schnitt entlang der Linie DD von Fig. 5;
Fig. 10 eine Ansicht in Blickrichtung E von Fig. 5;
Fig. 11 und 12 Schrägsichten der Einrichtung von Fig. 1 aus verschiedenen Blickrichtungen im geöffneten Zustand des Bandteils;
Fig. 13 eine schematische Darstellung der implantierten Einrichtung mit angeschlossenen Injektionsport.

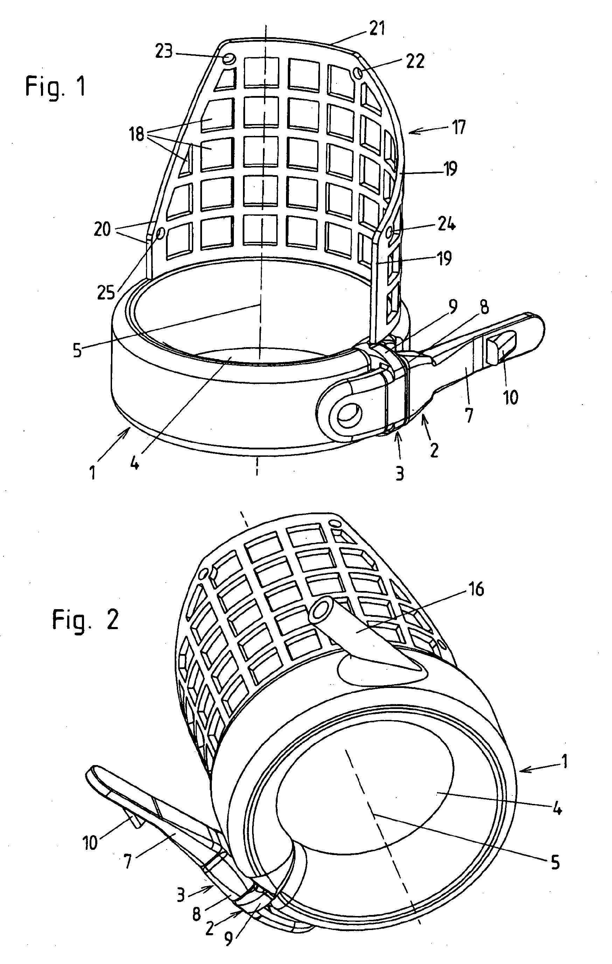

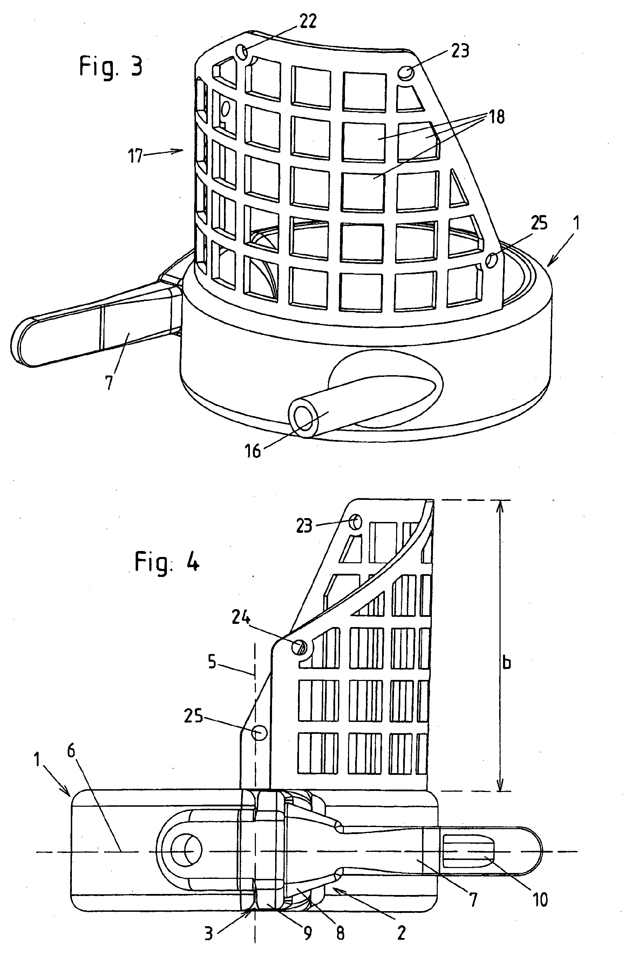

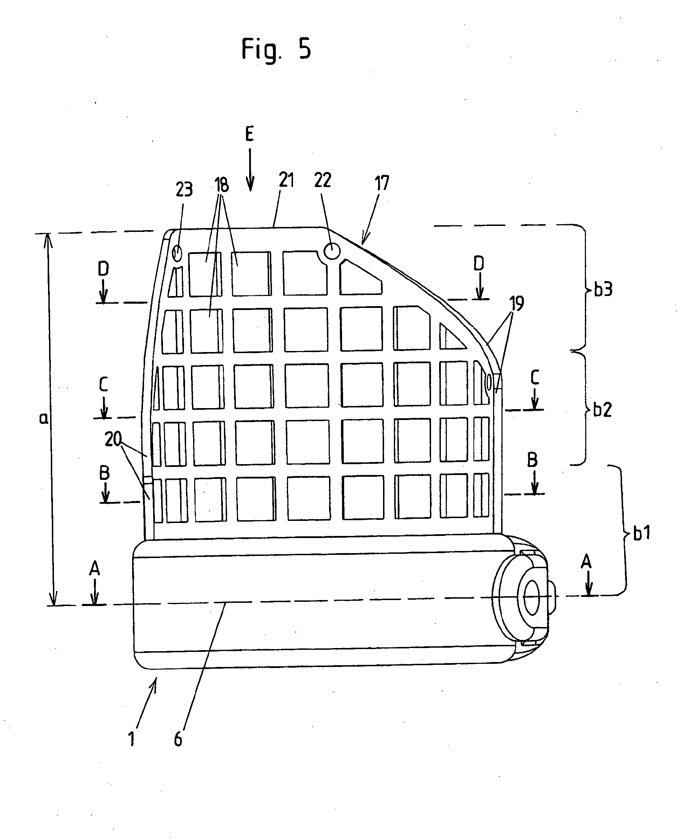

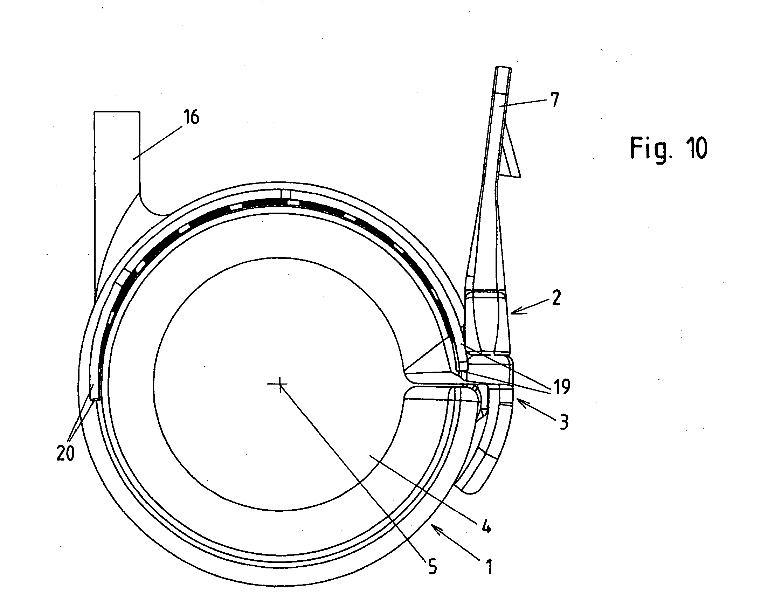

Ein Ausführungsbeispiel einer erfindungsgemäßen Einrichtung ist in den Fig. 1 bis 13 dargestellt. Die Einrichtung umfasst ein Bandteil 1, im Bereich von dessen beiden Enden erste und zweite Verschlussteile 2, 3 angeordnet sind, die eine Verschlusseinrichtung zum Verbindung der beiden Enden des Bandteils 1 bilden. Im geschlossenen Zustand der Verschlusseinrichtung (Fig. 1 bis 10 und 13) wird ein umfangsgeschlossener Ring ausgebildet, der eine Öffnung 4 mit einer zentralen Achse 5 umgibt. Das zu einem Ring verschlossene Bandteil 1 liegt in einer Mittelebene 6, die das Bandteil 1 mittig durchsetzt. Im Ausführungsbeispiel ist das Bandteil 1 spiegelsymmetrisch zur Mittelebene 6 ausgebildet. Die zentrale Achse 5, die die Öffnung 4 mittig durchsetzt, steht vorzugsweise rechtwinkelig zur Mittelebene 6.

Das geschlossene Bandteil 1 bildet insbesondere einen Kreisring, d.h. verläuft entlang einer Kreislinie.

Bei der gezeigten Ausführungsform der Verschlusseinrichtung besitzt das erste Verschlussteil 2 einen über das stirnseitige Ende des Bandteils 1 überstehenden Fortsatz 7 mit einem Rastvorsprung 8. Das zweite Verschlussteil 3 wird von einem in der Nähe des anderen stirnseitigen Endes des Bandteils 1 nach außen vorstehenden Steg 9 gebildet, der eine Einstecköffnung für den Fortsatz 7 aufweist. Der durch die Einstecköffnung durchgezogene Fortsatz 7 verrastet hinter dem Steg 9 mit seinem Rastvorsprung 8.

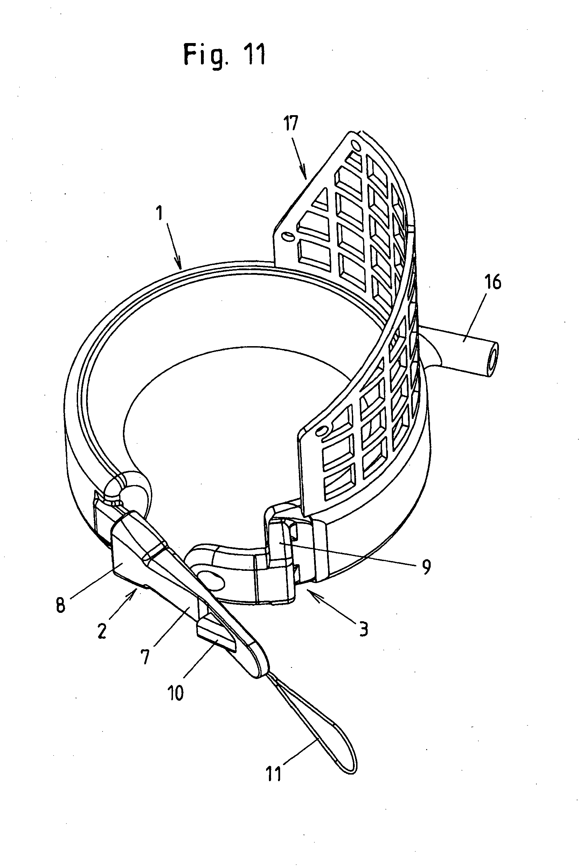

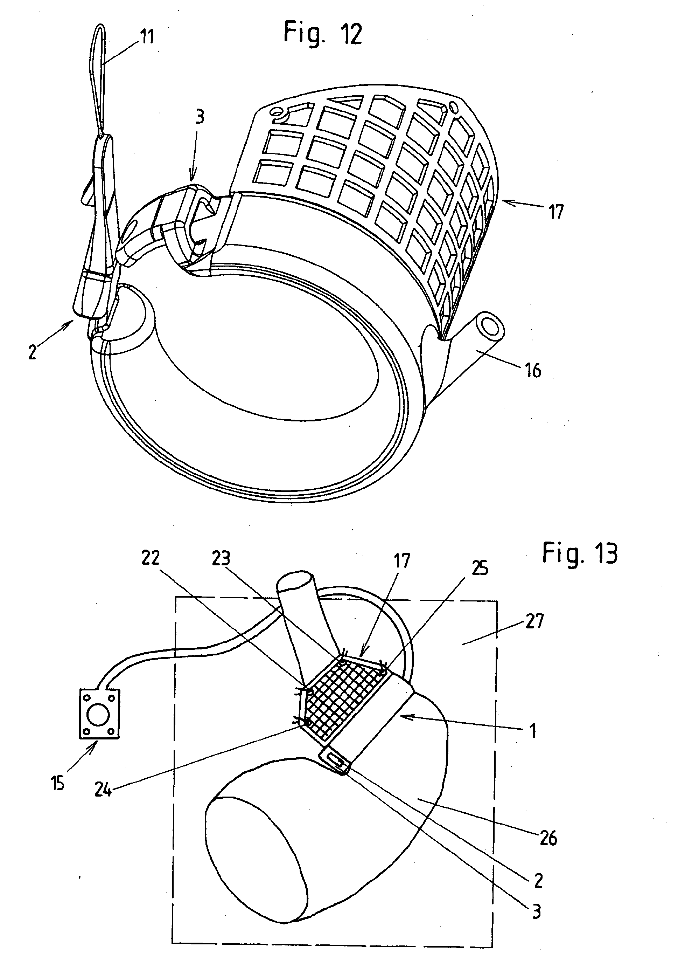

Mittels eines Vorrastvorsprungs (10) kann eine Vorraststellung im teilweise durchgezogenen Zustand des Fortsatzes 7 bereitgestellt werden. Zur Erleichterung des Durchziehens des Fortsatzes 7 kann eine Zugschlaufe 11 vorgesehen sein, die nur in den Fig. 11 und 12 dargestellt ist.

Auch in anderer Weise ausgebildete Verschlusseinrichtungen zum Verschließen des Bandteils 1 können vorgesehen sein.

Das Bandteil 1 besitzt im Inneren eine in seine Längsrichtung, vorzugsweise im Wesentlichen über seine gesamte Länge, verlaufende Hohlkammer 12, die mit einem Füllmedium befüllbar ist. Durch Einfüllen eines Füllmediums dehnt sich ein zur zentralen Achse 5 weisender innen liegender Bereich 13 des Bandteils 1 in Richtung zur zentralen Achse 5 aus, wodurch der Durchlassquerschnitt der Öffnung 4 eingestellt werden kann. Ein von der zentralen Achse 5 weggerichteter Bereich 14 des Bandteils 1 kann, beispielsweise durch eine Gewebeeinlage, eine gegenüber dem innen liegenden Bereich 13 geringere Dehnbarkeit aufweisen.

Zum Befüllen der Hohlkammer 12 kann in bekannter Weise ein Injektionsport 15 eingesetzt werden, der in Fig. 13 schematisch dargestellt ist und über einen Schlauch mit einem Anschlussröhrchen 16 des Bandteils 1 verbunden ist, dessen innerer Hohlraum in die Hohlkammer 12 mündet.

Das Bandteil 1 besteht zumindest im Bereich seiner äußeren Oberfläche aus einem biokompatiblen bzw. bioinerten Material, insbesondere Silikon oder Polyurethan. Vorzugsweise besteht das Bandteil 1, abgesehen von gegebenenfalls vorhandenen Verstärkungseinlagen und dem Anschlussröhrchen 16, insgesamt aus einem elastisch dehnbaren Material.

Mit dem Bandteil 1 ist ein Stützteil 17 verbunden, vorzugsweise durch Verklebung, welches vom Bandteil 1 seitlich absteht, d.h. es weist eine Erstreckung in Richtung der zentralen Achse 5 vom Bandteil 1 weg auf. Günstigerweise liegt die in Richtung der zentralen Achse 5 gemessene überstehende Breite b des Stützteils 17 im Bereich von 2cm bis 8cm.

Das Bandteil 1 und Stützteil 17 sind als vorgefertigte Einheit ausgebildet, d.h. bereits herstellerseitig miteinander verbunden. Vorzugsweise ist das Stützteil 17 im Bereich eines der Längsränder des Bandteils 1 an diesem angeklebt.

Das Stützteil 17 ist ein flächiges Gebilde, d.h. seine Dicke, die weniger als 4mm, vorzugsweise weniger als 2mm beträgt, ist wesentlich geringer als seine Länge und Breite.

Das Stützteil 17 besitzt eine Vielzahl von über seine Ausdehnung verteilten Durchtrittsöffnungen 18. Bevorzugterweise ist das Stützteil wie gezeigt netzartig bzw. gitterartig (=als Netz) ausgebildet. Auch eine Ausführung als elastische Folie mit oder ohne gestanzten Öffnungen ist denkbar. Die bevorzugte Größe der Durchtrittsöffnungen (die bei der Ausbildung als Netz oder als Maschenöffnungen bezeichnet werden können) liegt im Bereich von 1mm² bis 10mm².

Das Stützteil 17 ist elastisch und besteht zumindest im Bereich seiner äußeren Oberfläche, vorzugsweise durchgehend, aus einem biokompatiblen bzw. bioinerten Material, insbesondere aus einem Elastomer, beispielsweise aus Silikon oder Polyurethan. Die Härte des Materials des Stützteils 17 liegt günstigerweise im Bereich von 10 bis 60 Shore A.

Im zu einem Ring geschlossenen Zustand des Bandteils 1 erstreckt sich das Stützteil 17 nur über einen Teil des vollständigen Umfangs um die zentrale Achse 5. Genauer gesagt erstreckt sich das Stützteil 17 in jedem parallel zur Mittelebene 6 liegenden Querschnitt durch das Stützteil 17 über weniger als 270° um die zentrale Achse, vorzugsweise um weniger als 225°.

Im gezeigten Ausführungsbeispiel ist hierbei im Bereich b3 des Stützteils 17, in welchem der Abstand des Stützteils 17 von der Mittelebene 6 mehr als zwei Drittel des maximalen Abstands a des Stützteils 17 von der Mittelebene 6 beträgt, kleiner, vorzugsweise um mindestens 10% kleiner, als die größte Erstreckung des Stützteils 17 um die zentrale Achse 5 im Bereich b1 des Stützteils 17, in welchem der Abstand des Stützteils 17 weniger als ein Drittel des maximalen Abstands a des Stützteils 17 von der Mittelebene 6 beträgt.

Im gezeigten Ausführungsbeispiel ist im Bereich b2 des Stützteils 17, in welchem der Abstand des Stützteils 17 von der Mittelebene 6 mehr als ein Drittel und weniger als zwei Drittel des maximalen Abstands a des Stützteils 17 von der Mittelebene 6 beträgt, die größte Erstreckung des Stützteils 17 um die zentrale Achse 5 kleiner, vorzugsweise mindestens 10% kleiner, als die größte Erstreckung des Stützteils 17 um die zentrale Achse 5 im Bereich b1 des Stützteils, in welchem der Abstand des Stützteils 17 von der Mittelebene 6 weniger als ein Drittel des maximalen Abstands a des Stützteils 17 von der Mittelebene 6 beträgt.

Weiters ist im gezeigten Ausführungsbeispiel im Bereich b3 des Stützteils 17, in welchem der Abstand des Stützteils 17 von der Mittelebene 6 mehr als zwei Drittel des maximalen Abstands a beträgt, die größte Erstreckung des Stützteils 17 um die zentrale Achse 5 kleiner, vorzugsweise um mindestens 10% kleiner, als die größte Erstreckung des Stützteils 17 um die zentrale Achse 5 im Bereich b2 des Stützteils 17, in welchem der Abstand des Stützteils 17 von der Mittelebene 6 mehr als ein Drittel und weniger als zwei Drittel des maximalen Abstands beträgt.

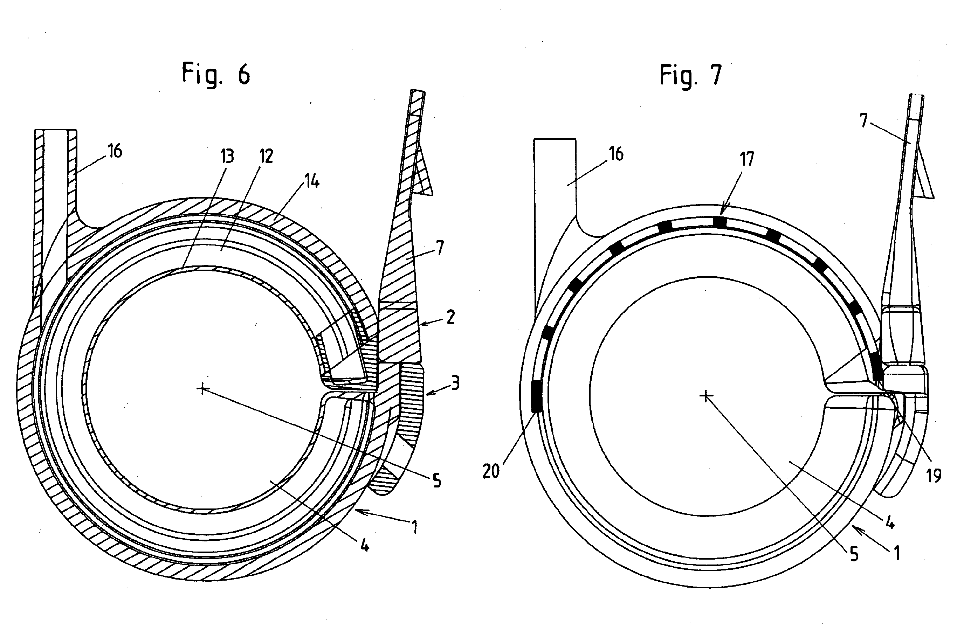

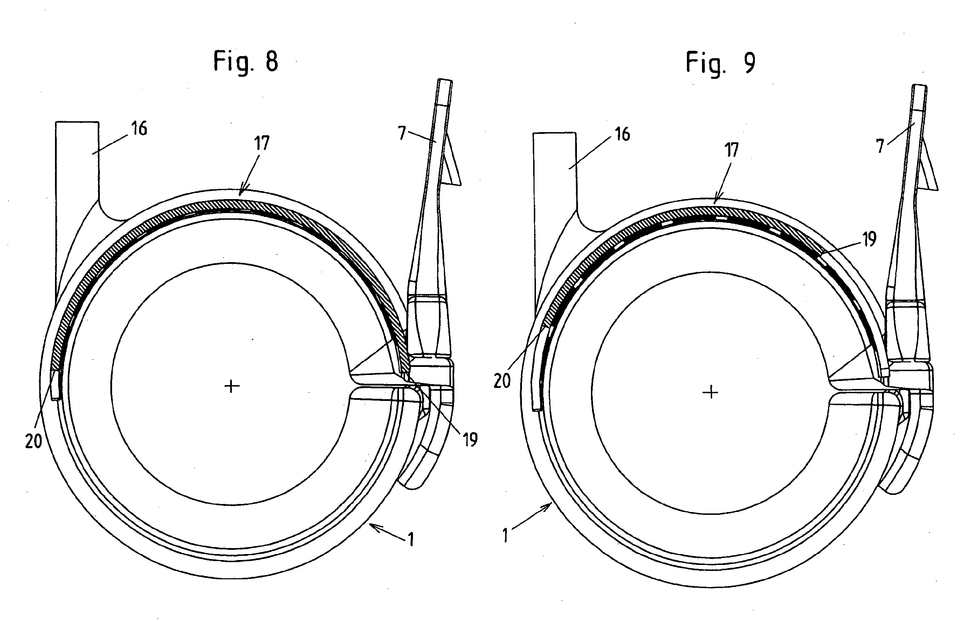

Die Fig. 7 zeigt einen im Bereich b1 liegenden Querschnitt. Die Fig. 8 zeigt einen im Bereich b2 liegenden Querschnitt. Die Fig. 9 zeigt einen im Bereich b3 liegenden Querschnitt durch das Stützteil 17.

Die Verringerung der Erstreckung des Stützteils 17 um die zentrale Achse 5 mit zunehmendem Abstand von der Mittelebene 6 wird durch mindestens einen schräg verlaufenden Abschnitt eines der Längsränder 19, 20 des Stützteils 17 ausgebildet, welche die Erstreckung des Stützteils 17 um die zentrale Achse 5, d.h. in die Umfangsrichtung, begrenzen. Auch ein gestufter Verlauf wäre denkbar und möglich.

Die Erstreckung des Stützteils 17 in Richtung der zentralen Achse 5 wird am vom Bandteil 1 abgelegenen Ende des Stützteils 17 durch einen Seitenrand 21 des Stützteils 17 begrenzt, der hier in einer parallel zur Mittelebene 6 liegenden Ebene liegt. Auch ein zumindest abschnittsweise schräger oder abgestufter Verlauf des Seitenrandes 21 ist denkbar und möglich.

Das Stützteil 17 ist lateral zum Magen an der Hinterwand der Bauchhöhle befestigbar, vorzugsweise durch Vernähen. Hierzu sind in den Bereichen des Übergangs zwischen dem Seitenrand 21 des Stützteils 17 und den Längsrändern 19, 20 des Stützteils 17 Nähösen 22, 23 des Stützteils 17 vorgesehen (die schräg verlaufenden Randbereiche des Stützteils 17 werden hier den Längsrändern 19, 20 zugeordnet, da ihre Steigung gegenüber der zentralen Achse 5 mindestens 45° beträgt).

Weiters sind günstigerweise im Bereich des Übergangs zwischen den schräg verlaufenden Abschnitten der Längsränder 19, 20 und den parallel zur zentralen Achse 5 verlaufenden Abschnitten der Längsränder 19, 20 Nähösen 24, 25 ausgebildet.

Zusätzliche Nähösen, insbesondere im Bereich der Längsränder 19, 20 können vorgesehen sein.

Um die vom Magen auf das Stützteil 17 ausgeübten Kräfte aufnehmen zu können, ist das Stützteil 17 im Bereich der Nähösen 22-25 vorzugsweise verstärkt ausgebildet, beispielsweise durch in diesen Bereichen vorgesehene Einlagen.

Die Umfangserstreckung des Stützteils 17 an der Stelle, an der es vom Bandteil 1 ausgeht, also am Beginn seiner vom Bandteil 1 abstehenden Erstreckung, liegt vorzugsweise im Bereich von 4,5cm bis 10cm. Die Umfangserstreckung des Stützteils 17 an seinem vom Bandteil 1 entfernt gelegenen Ende liegt vorzugsweise im Bereich von 2,5cm bis 7cm.

Im gezeigten Ausführungsbeispiel ist der Krümmungsradius des Stützteils 17 um die zentrale Achse 5 über seine Erstreckung in Richtung der zentralen Achse 5 konstant und entspricht dem Krümmungsradius des Bandteils 1, jeweils bezogen auf den zu einem Ring verschlossenen Zustand des Bandteils 1. Der Krümmungsradius des Stützteils 17 im Bereich seines vom Bandteil 1 entfernt gelegenen Endes könnte auch geringer sein als der Krümmungsradius im Bereich seines mit dem Bandteil 1 verbundenen Endes, beispielsweise um mindestens 30% geringer.

Denkbar und möglich wäre auch eine bauchige Ausbildung des Stützteils 17, wobei das Stützteil 17 in einem mittleren Bereich seiner Erstreckung bezogen auf die Richtung der zentralen Achse 5, zumindest über einen Teil seiner Umfangserstreckung um die zentrale Achse 5, einen größeren Abstand von der zentralen Achse 5 aufweist als in Bereichen, die einen geringeren Abstand bzw. einen größeren Abstand vom Bandteil 1 aufweisen als der mittlere Bereich. Damit ist der Krümmungsradius des Stützteils 17 im mittleren Bereich, zumindest über einen Teil seiner Umfangserstreckung um die zentrale Achse 5, kleiner als in den beidseitig anschließenden Bereichen. Dadurch kann eine gute Anpassung an die Magenform erreicht werden.

Der Bereich der Längserstreckung des Bandteils 1, über welchen das Stützteil 17 seitlich von ihm absteht, schließt an das Ende des Bandteils 1 an, an welchem das zweite Verschlussteil 3 angeordnet ist. Anschließend an das andere Ende des Bandteils 1, an welchem das erste Verschlussteil 2 angeordnet ist, ist ein Abschnitt der Längserstreckung des Bandteils 1 ohne seitlich von diesem abstehendes Stützteil 17 vorhanden.

Fig. 13 zeigt schematisch den am Magen 26 eines Patienten angelegten Zustand der erfindungsgemäßen Einrichtung. Das Bandteil 1 ist um den Magen 26 gelegt und mittels der Verschlusseinrichtung 2, 3 zu einem Ring geschlossen. Um das Bandteil 1 um den Magen zu legen ist zuvor ein zwischen der Magenhinterwand und der Hinterwand der Bauchhöhle verlaufender Tunnel (Kanal) präpariert worden, durch den das erste Ende des noch offenen Bandteils 1 gesteckt worden ist, an welches der Abschnitt des Bandteils 1 anschließt, der ohne ein von ihm seitlich abstehendes Stützteil 17 ausgebildet ist.

Nachdem das Bandteil 1 um den Magen 26 gelegt und verschlossen worden ist, liegt das Stützteil 17 im proximal des Bandteils 1 liegenden Bereich an der anterioren ventralen Magenwand an. Nunmehr wird das Stützteil 17 lateral zum Magen und beidseitig von diesem mit der Hinterwand 27 der Bauchhöhle, von der in Fig. 13 ein Abschnitt schematisch dargestellt ist, verbunden, insbesondere durch Vernähen durch die Nähösen, 22-25.

Auch der Bereich des ösophagealen-gastralen Übergangs kann vom Stützteil 17 abgestützt sein.

Auf der dem Stützteil 17 gegenüberliegenden Seite des Bandteils 1 könnte grundsätzlich ein weiteres Stützteil vom Bandteil 1 abstehen. Dieses würde dann in einem distal zum Bandteil 1 liegenden Bereiches des Magens außen am Magen anliegen. Ein solches weiteres, insbesondere netzartiges, Stützteil könnte von einem separaten Teil gebildet werden oder einteilig mit dem Stützteil 17 ausgebildet sein.

### Legende zu den Hinweisziffern:

- 1: Bandteil
- 2: erstes Verschlussteil
- 3: zweites Verschlussteil
- 4: Öffnung
- 5: zentrale Achse
- 6: Mittelebene
- 7: Fortsatz
- 8: Rastvorsprung
- 9: Steg
- 10: Vorrastvorsprung
- 11: Zugschlaufe
- 12: Hohlkammer
- 13: innen liegender Bereich
- 14: außen liegender Bereich
- 15: Injektionsport
- 16: Anschlussröhrchen
- 17: Stützteil
- 18: Durchtrittsöffnung
- 19: Längsrand
- 20: Längsrand
- 21: Seitenrand
- 22: Nähöse
- 23: Nähöse
- 24: Nähöse
- 25: Nähöse
- 26: Magen
- 27: Hinterwand

## Patentansprüche

1. Einrichtung zur Behandlung von Fettleibigkeit, umfassend ein ringförmig um den Magen legbares Bandteil (1) mit einer Verschlusseinrichtung (2, 3), mit der das Bandteil (1) zu einem umfangsgeschlossenen Ring verschließbar ist, der eine Öffnung (4) mit einer zentralen Achse (5) aufweist, und ein mit dem Bandteil (1) verbundenes und von diesem auf einer Seite abstehendes Stützteil (17) zum Abstützen eines gegenüber dem angelegten Bandteil (1) proximal liegenden Magenbereichs, **dadurch gekennzeichnet, dass** sich das Stützteil (17) in der Schnittebene eines jeden durch das Stützteil (17) verlaufenden Querschnitts, der parallel zu einer Mittelebene (6) liegt, in welcher das zu einem Ring verschlossene Bandteil (1) liegt und welche mittig durch das Bandteil (1) verläuft, über weniger als 270° um die zentrale Achse (5) erstreckt.

2. Einrichtung nach Anspruch 1, **dadurch gekennzeichnet, dass** das Bandteil (1) einen ersten Abschnitt seiner Längserstreckung aufweist, der an ein erstes Ende des Bandteils (1) anschließt und dessen Länge zumindest ein Drittel der gesamten Länge des Bandteils (1) beträgt, vorzugsweise zumindest zwei Fünftel der gesamten Länge des Bandteils (1) beträgt, und einen zweiten Abschnitt seiner Längserstreckung aufweist, der an das zweite Ende des Bandteils (1) anschließt und dessen Länge die restliche Länge des Bandteils (1) beträgt, wobei das Stützteil (17) nur über den zweiten Abschnitt der Längserstreckung des Bandteils (1) vom Bandteil (1) auf einer Seite absteht.

3. Einrichtung nach Anspruch 1 oder 2, **dadurch gekennzeichnet, dass** im Bereich (b3) des Stützteils (17), der in einem Abstand des Stützteils (17) von der Mittelebene (6) liegt, welcher mehr als zwei Drittel des maximalen Abstands (a) eines Seitenrands (21) des Stützteils (17) von der Mittelebene (6) beträgt, die größte Erstreckung des Stützteils (17) um die zentrale Achse (5) kleiner ist, vorzugsweise um mindestens 10% kleiner ist, als die größte Erstreckung des Stützteils (17) um die zentrale Achse (5) im Bereich (b1) des Stützteils (17), der in einem Abstand von der Mittelebene (6) liegt, welcher weniger als ein Drittel des maximalen Abstands (a) des Seitenrands (21) des Stützteils (17) von der Mittelebene (6) beträgt.

4. Einrichtung nach Anspruch 3, **dadurch gekennzeichnet, dass** im Bereich (b2) des Stützteils (17), der in einem Abstand des Stützteils (17) von der Mittelebene (6) liegt, welcher mehr als ein Drittel und weniger als zwei Drittel des maximalen Abstands (a) des Seitenrands (21) des Stützteils (17) von der Mittelebene (6) beträgt, die größte Erstreckung des Stützteils (17) um die zentrale Achse (5) kleiner ist, vorzugsweise um mindestens 10% kleiner ist, als die größte Erstreckung des Stützteils (17) um die zentrale Achse (5) im Bereich (b1) des Stützteils (17), der in einem Abstand von der Mittelebene (6) liegt, welcher weniger als ein Drittel des maximalen Abstands (a) des Seitenrands (21) des Stützteils (17) von der Mittelebene (6) beträgt.

5. Einrichtung nach Anspruch 3 oder 4, **dadurch gekennzeichnet, dass** im Bereich (b3) des Stützteils (17), der in einem Abstand des Stützteils (17) von der Mittelebene (6) liegt, welcher mehr als zwei Drittel des maximalen Abstands (a) des Seitenrands (21) des Stützteils (17) von der Mittelebene (6) beträgt, die größte Erstreckung des Stützteils (17) um die zentrale Achse (5) kleiner, vorzugsweise um mindestens 10% kleiner, ist als die größte Erstreckung des Stützteils (17) um die zentrale Achse (5) im Bereich (b2) des Stützteils (17), der in einem Abstand von der Mittelebene (6) liegt, welcher mehr als ein Drittel und weniger als zwei Drittel des maximalen Abstands (a) des Seitenrands (21) des Stützteils (17) von der Mittelebene (6) beträgt.

6. Einrichtung nach einem der Ansprüche 1 bis 5, **dadurch gekennzeichnet, dass** das Stützteil (17) eine Vielzahl von über seine Ausdehnung verteilten Durchtrittsöffnungen (19) aufweist, wobei die Summe der Flächen der Durchtrittsöffnungen (19) vorzugsweise einen Großteil der Gesamtfläche des Stützteils (17) ausmacht.

7. Einrichtung nach Anspruch 6, **dadurch gekennzeichnet, dass** das Stützteil (17) netzartig ausgebildet ist.

8. Einrichtung nach einem der Ansprüche 1 bis 7, **dadurch gekennzeichnet, dass** das Stützteil (17) am Bandteil (1) angeklebt ist, vorzugsweise im Bereich eines der Längsränder des Bandteils (1).

9. Einrichtung nach einem der Ansprüche 1 bis 8, **dadurch gekennzeichnet, dass** das Stützteil (17) im Bereich der Übergänge zwischen den Längsrändern (19, 20) des Stützteils (17), die die Erstreckung des Stützteils (17) um die zentrale Achse (5) begrenzen, und dem Seitenrand (21) des Stützteils (17), der am vom Bandteil (1) entfernten Ende des Stützteils (17) die Erstreckung des Stützteils in Richtung der zentralen Achse (5) begrenzt, mit der Hinterwand der Bauchhöhle verbindbar, vorzugsweise vernähbar, ausgebildet ist.

10. Einrichtung nach einem der Ansprüche 1 bis 9, **dadurch gekennzeichnet, dass** das Stützteil (17) zur Vernähung mit der Hinterwand (27) der Bauchhöhle beidseitig des Magens (26) Nähösen (22, 23, 24, 25) aufweist.

## Claims

1. A device for the treatment of obesity, comprising a band part (1), able to be placed annularly around the stomach, with a closing device (2, 3) with which the band part (1) can be closed to form a circumferentially-closed ring which has an opening (4) with a central axis (5), and comprising a supporting part (17), connected to the band part (1) and projecting therefrom on one side, to support a stomach region lying proximally with respect to the applied band part (1), **characterised in that** in the sectional plane of each crosssection, running through the supporting part (17), lying parallel to a centre plane (6) in which there lies the band part (1) closed to form a ring and which runs centrally through the band part (1), the supporting part (17) extends over less than 270° about the central axis (5).

2. A device according to claim 1, **characterised in that** the band part (1) has a first longitudinal-extension portion which adjoins a first end of the band part (1) and whose length is at least one third of the entire length of the band part (1), preferably at least two fifths of the entire length of the band part (1), and has a second longitudinal-extension portion which adjoins the second end of the band part (1) and whose length amounts to the remaining length of the band part (1), wherein the supporting part (17) only projects on one side from the band part (1) over the second portion of the longitudinal extension of the band part (1).

3. A device according to claim 1 or 2, **characterised in that** in the region (b3), of the supporting part (17), lying at a distance of the supporting part (17) from the centre plane (6) of more than two thirds of the maximum distance (a) of a lateral edge (21) of the supporting part (17) from the centre plane (6), the greatest extension of the supporting part (17) about the central axis (5) is smaller, preferably smaller by at least 10%, than the greatest extension of the supporting part (17) about the central axis (5) in the region (b1), of the supporting part (17), lying at a distance from the centre plane (6) of less than one third of the maximum distance (a) of the lateral edge (21) of the supporting part (17) from the centre plane (6).

4. A device according to claim 3, **characterised in that** in the region (b2), of the supporting part (17), lying at a distance of the supporting part (17) from the centre plane (6) of more than one third and less than two thirds of the maximum distance (a) of the lateral edge (21) of the supporting part (17) from the centre plane (6), the greatest extension of the supporting part (17) about the central axis (5) is smaller, preferably smaller by at least 10%, than the greatest extension of the supporting part (17) about the central axis (5) in the region (b1), of the supporting part (17), lying at a distance from the centre plane (6) of less than one third of the maximum distance (a) of the lateral edge (21) of the supporting part (17) from the centre plane (6).

5. A device according to claim 3 or 4, **characterised in that** in the region (b3), of the supporting part (17), lying at a distance of the supporting part (17) from the centre plane (6) of more than two thirds of the maximum distance (a) of the lateral edge (21) of the supporting part (17) from the centre plane (6), the greatest extension of the supporting part (17) about the central axis (5) is smaller, preferably smaller by at least 10%, than the greatest extension of the supporting part (17) about the central axis (5) in the region (b2), of the supporting part (17), lying at a distance from the centre plane (6) of more than one third and less than two thirds of the maximum distance (a) of the lateral edge (21) of the supporting part (17) from the centre plane (6).

6. A device according to any one of claims 1 to 5, **characterised in that** the supporting part (17) has a plurality of openings (19[sic]) distributed over its expanse, wherein the sum of the areas of the openings (19) preferably represents a majority of the total area of the supporting part (17).

7. A device according to claim 6, **characterised in that** the supporting part (17) is meshed.

8. A device according to any one of claims 1 to 7, **characterised in that** the supporting part (17) is stuck to the band part (1), preferably in the region of one of the longitudinal edges of the band part (1).

9. A device according to any one of claims 1 to 8, **characterised in that** in the region of the transitions between the longitudinal edges (19, 20) of the supporting part (17), which bound the extension of the supporting part (17) about the central axis (5), and the lateral edge (21) of the supporting part (17), which at the end of the supporting part (17) remote from the band part (1) bounds the extension of the supporting part in the direction of the central axis (5), the supporting part (17) is connectable, preferably suturable, to the rear wall of the abdominal cavity.

10. A device according to any one of claims 1 to 9, **characterised in that** the supporting part (17) has suturing eyes (22, 23, 24, 25) on both sides of the stomach (26), for the purpose of suturing to the rear wall (27).

## Revendications

1. Dispositif de traitement de l'obésité comportant une partie en forme de bande annulaire (1) pouvant être positionnée autour de l'estomac, et munie d'un dispositif de fermeture (2, 3) permettant de fermer la partie en forme de bande (1) pour obtenir un anneau fermé sur sa périphérie, qui comporte une ouverture (4) avec un axe central (5), ainsi qu'une partie d'appui (17) reliée à la partie en forme de bande (1) et prolongeant celle-ci d'un côté pour s'appuyer contre une zone de l'estomac proximale par rapport à la partie en forme de bande (1),
**caractérisée en ce que**
la partie d'appui (17) s'étend sur moins de 270° autour de l'axe central (5) dans le plan de coupe d'une section transversale de cette partie d'appui (17) parallèle au plan médian (6) de la partie en forme de bande (1) fermée en un anneau, orienté selon une position médiane au travers de la partie en forme de bande (1).

2. Dispositif conforme à la revendication 1,
**caractérisée en ce que**
la partie en forme de bande (1) comporte un premier tronçon de son étendue longitudinale adjacent à une première extrémité de celle-ci et dont la longueur est d'au moins un tiers de la longueur totale de cette partie en forme de bande (1), de préférence d'au moins deux cinquième de la longueur totale de cette partie en forme de bande (1) et un second tronçon de son étendue longitudinale adjacent à la seconde extrémité de celle-ci et dont la longueur correspond à la longueur résiduelle de cette partie en forme de bande (1), la partie d'appui (17) ne s'écartant d'un côté de la partie en forme de bande (1) que sur le second tronçon de l'étendue longitudinale de cette partie en forme de bande (1).

3. Dispositif conforme à la revendication 1 ou 2,
**caractérisée en ce que**
dans la zone (b3) de la partie d'appui (17) qui est située à une distance du plan médian (6) qui est égale à plus des deux tiers de la distance maximum (a) d'un bord latéral (21) de la partie d'appui (17) du plan médian (6), la plus grande étendue de la partie d'appui (17) autour de l'axe central (5) est plus petite, de préférence plus petite d'au moins 10 % que la plus grande étendue de la partie d'appui (17) autour de l'axe central (5) dans la zone (b1) de cette partie d'appui (17) qui est située à une distance du plan médian (6) qui est égale à moins d'un tiers de la distance maximum (a) du bord latéral (21) de la partie d'appui (17) du plan médian (6).

4. Dispositif conforme à la revendication 3,
**caractérisée en ce que**
dans la zone (b2) de la partie d'appui (17) qui est située à une distance du plan médian (6) qui est égale à plus d'un tiers et à moins des deux tiers de la distance maximum (a) du bord latérale (21) de la partie d'appui (17) du plan médian (6), la plus grande étendue de la partie d'appui (17) autour de l'axe central (5) est plus petite, de préférence plus petite d'au moins 10% que la plus grande étendue de la partie d'appui (17) autour de l'axe central (5) dans la zone (b1) de cette partie d'appui (17) qui est située à une distance du plan médian (6) qui est égale à moins d'un tiers de la distance maximum (a) du bord latéral (21) de la partie d'appui (17) du plan médian (6).

5. Dispositif conforme à la revendication 3 ou 4,
**caractérisée en ce que**
dans la zone (b3) de l'élément d'appui (17) qui est située à une distance du plan médian (6) qui est égale à plus des deux tiers de la distance maximum (a) du bord latéral (21) de la partie d'appui (17) du plan médian (6), la plus grande étendue de la partie d'appui (17) autour de l'axe central (5) est plus petite, de préférence plus petite d'au moins 10% que la plus grande étendue de la partie d'appui (17) autour de l'axe central (5) dans la zone (b2) de cette partie d'appui (17) qui est située à une distance du plan médian (6) qui est égale à plus d'un tiers et à moins des deux tiers de la distance maximum (a) du bord latéral (21) de la partie d'appui (17) du plan médian (6).

6. Dispositif conforme à l'une des revendications 1 à 5,
**caractérisée en ce que**
la partie d'appui (17) comporte plusieurs ouvertures traversantes (18) réparties sur son étendue, la somme des surfaces des ouvertures traversantes (18) représentant de préférence une grande partie de la surface totale de la partie d'appui (17).

7. Dispositif conforme à la revendication 6,
**caractérisée en ce que**
la partie d'appui (17) est réalisée en forme d'un réseau.

8. Dispositif conforme à l'une des revendications 1 à 7,
**caractérisée en ce que**
la partie d'appui (17) est collée à la partie en forme de bande (1) de préférence dans la zone de l'un des bords longitudinaux de cette partie en forme de bande (1).

9. Dispositif conforme à l'une des revendications 1 à 8,
**caractérisée en ce que**
la partie d'appui (17) est réalisée dans la zone de la transition entre les bords longitudinaux (19, 20) de cette partie d'appui (17) qui limitent l'étendue de celle-ci autour de l'axe central (5) et le bord latéral (21) de la partie d'appui (17) qui limite l'étendue de la partie d'appui en direction de l'axe central (5) à l'extrémité de la partie d'appui (17) éloignée de la partie en forme de bande (1) de manière à pouvoir être reliée de préférence suturée à la paroi arrière de la cavité abdominale.

10. Dispositif conforme à l'une des revendications 1 à 9,
**caractérisée en ce que**
la partie d'appui (17) comporte des oeillets de suture (22, 23, 24, 25) permettant sa suture avec la paroi interne (27) de la cavité abdominale de chaque côté de l'estomac (26).
